# EUROPEAN PATENT APPLICATION

(11) **EP 3 431 596 A1**
(43) Date of publication of application: **23.01.2019**
(21) Application number: 17766719.3
(22) Date of filing: 15.03.2017
(51) Int. Cl.: C12N 15/09, C12N 9/38, C12N 9/50, C12N 9/60, C12Q 1/34, C12Q 1/37

(54) **PROTEASE B, AND LACTASE SOLUTION UTILIZING PROPERTIES THEREOF AND METHOD FOR MANUFACTURING SAME**

(30) Priority: 16.03.2016 JP 2016053138
(71) Applicant: Godo Shusei Co., Ltd., Tokyo 104-0061 (JP)
(72) Inventor: SUGAWARA, Asami, Matsudo-shi Chiba 271-0064 (JP); YOSHIKAWA, Jun, Matsudo-shi Chiba 271-0064 (JP)
(74) Representative: Hoffmann Eitle
(86) International application number: PCT/JP2017/010356
(87) International publication number: WO 2017/159723

(57) **Abstract**

[Problem to be Solved] To provide a lactase solution with good heat stability as desired by identifying a factor to influence heat stability in a lactase product and using the factor as an index.

[Solution] Proteinase B having the following enzymatic properties, and a lactase solution containing less than 11.5 ng of the proteinase B.
1) having an optimum temperature of about 40°C,
2) having an optimum pH of about 8.0,
3) being stable at pH 5.0 to 8.0,
4) having high substrate specificity to FITC-casein and lactase,
5) having a neutral lactase-fragmenting action, and
6) having a molecular weight of about 29,700 to 30,000 (by SDS-PAGE).

SEQUENCE LISTING

<110>  GODO SHUSEI CO., LTD

<120>  PROTEINASE B AND LACTASE SOLUTION UTILIZING PROPERTY OF
       PROTEINASE B AND METHOD FOR MANUFACTURING THE SAME

<130>  FP0285PCT

<160>  9

<170>  PatentIn version 3.5

<210>  1
<211>  298
<212>  PRT
<213>  Kluyveromyces lactis

<400>  1

Ser Phe Asn Thr Gln Asn Ser Ala Pro Trp Gly Leu Ala Arg Ile Ser
1               5                   10                  15

His Arg Glu Lys Leu Asn Leu Gly Ser Phe Asn Lys Tyr Leu Tyr Asp
            20                  25                  30

Asp Asp Ala Gly Lys Gly Val Thr Ala Tyr Val Val Asp Thr Gly Val
        35                  40                  45

Asn Val Asn His Lys Asp Phe Asp Gly Arg Ala Val Trp Gly Lys Thr
    50                  55                  60

Ile Pro Lys Asp Asp Pro Asp Val Asp Gly Asn Gly His Gly Thr His
65                  70                  75                  80

Cys Ala Gly Thr Ile Gly Ser Val His Tyr Gly Val Ala Lys Asn Ala
                85                  90                  95

Asp Ile Val Ala Val Lys Val Leu Arg Ser Asn Gly Ser Gly Thr Met
            100                 105                 110

Ser Asp Val Val Lys Gly Val Glu Tyr Val Ala Glu Ala His Lys Lys
        115                 120                 125

Ala Val Glu Glu Gln Lys Lys Gly Phe Lys Gly Ser Thr Ala Asn Met
    130                 135                 140

Ser Leu Gly Gly Gly Lys Ser Pro Ala Leu Asp Leu Ala Val Asn Ala
145                 150                 155                 160

Ala Val Lys Ala Gly Val His Phe Ala Val Ala Ala Gly Asn Glu Asn
                165                 170                 175

Gln Asp Ala Cys Asn Thr Ser Pro Ala Ala Ala Glu Asn Ala Ile Thr
            180                 185                 190

Val Gly Ala Ser Thr Leu Ser Asp Glu Arg Ala Tyr Phe Ser Asn Trp
        195                 200                 205

Gly Lys Cys Val Asp Ile Phe Gly Pro Gly Leu Asn Ile Leu Ser Thr
    210                 215                 220

Tyr Ile Gly Ser Asp Thr Ala Thr Ala Thr Leu Ser Gly Thr Ser Met
225                 230                 235                 240

Ala Thr Pro His Val Val Gly Leu Leu Thr Tyr Phe Leu Ser Leu Gln
                245                 250                 255

Pro Asp Ala Asp Ser Glu Tyr Phe His Ala Ala Gly Gly Ile Thr Pro
            260                 265                 270

Ser Gln Leu Lys Lys Lys Leu Ile Asp Phe Ser Thr Lys Asn Val Leu
        275                 280                 285

Ser Asp Leu Pro Glu Asp Thr Val Asn Tyr
    290                 295

<210>  2
<211>  561
<212>  PRT
<213>  Kluyveromyces lactis

<400>  2

Met Lys Phe Glu Asn Thr Leu Leu Thr Ile Thr Ala Leu Ser Thr Val
1               5                   10                  15

Ala Thr Ala Leu Val Ile Pro Glu Val Asn Arg Glu Asn Lys His Gly
            20                  25                  30

Asp Lys Ser Val Ala Ile Lys Asp His Ala Ser Ser Asp Leu Asp Lys
        35                  40                  45

Pro Gln His His Ala Asn Gly Lys Ala Arg Ser Lys Ser Arg Gly Arg
    50                  55                  60

Cys Ala Asp Ser Lys Lys Phe Asp Lys Leu Arg Pro Val Asp Asp Ala
65                  70                  75                  80

Ser Ala Ile Leu Ala Pro Leu Ser Thr Val Asn Asp Ile Ala Asn Lys
                85                  90                  95

Ile Pro Asn Arg Tyr Ile Ile Val Phe Lys Lys Asp Ala Ser Ala Asp
        100                 105                 110

Glu Val Lys Phe His Gln Glu Leu Val Ser Val Glu His Ala Lys Ala
        115                 120                 125

Leu Gly Ser Leu Ala Asp Asn Asp Pro Phe Phe Thr Ala Thr Ser Gly
    130                 135                 140

Glu His Ser Glu Phe Gly Val Lys Ala His Ser Leu Glu Gly Gly Ile
145                 150                 155                 160

Gln Asp Ser Phe Asp Ile Ala Gly Ser Leu Ser Gly Tyr Val Gly Tyr
                165                 170                 175

Phe Thr Lys Glu Val Ile Asp Phe Ile Arg Arg Ser Pro Leu Val Glu
            180                 185                 190

Phe Val Glu Glu Asp Ser Met Val Phe Ser Asn Ser Phe Asn Thr Gln
        195                 200                 205

Asn Ser Ala Pro Trp Gly Leu Ala Arg Ile Ser His Arg Glu Lys Leu
    210                 215                 220

Asn Leu Gly Ser Phe Asn Lys Tyr Leu Tyr Asp Asp Asp Ala Gly Lys
225                 230                 235                 240

Gly Val Thr Ala Tyr Val Val Asp Thr Gly Val Asn Val Asn His Lys
                245                 250                 255

Asp Phe Asp Gly Arg Ala Val Trp Gly Lys Thr Ile Pro Lys Asp Asp
            260                 265                 270

Pro Asp Val Asp Gly Asn Gly His Gly Thr His Cys Ala Gly Thr Ile
        275                 280                 285

Gly Ser Val His Tyr Gly Val Ala Lys Asn Ala Asp Ile Val Ala Val
    290                 295                 300

Lys Val Leu Arg Ser Asn Gly Ser Gly Thr Met Ser Asp Val Val Lys
305                 310                 315                 320

Gly Val Glu Tyr Val Ala Glu Ala His Lys Lys Ala Val Glu Glu Gln
                325                 330                 335

Lys Lys Gly Phe Lys Gly Ser Thr Ala Asn Met Ser Leu Gly Gly Gly
            340                 345                 350

Lys Ser Pro Ala Leu Asp Leu Ala Val Asn Ala Ala Val Lys Ala Gly
        355                 360                 365

Val His Phe Ala Val Ala Ala Gly Asn Glu Asn Gln Asp Ala Cys Asn
    370                 375                 380

Thr Ser Pro Ala Ala Ala Glu Asn Ala Ile Thr Val Gly Ala Ser Thr
385                 390                 395                 400

Leu Ser Asp Glu Arg Ala Tyr Phe Ser Asn Trp Gly Lys Cys Val Asp
                405                 410                 415

Ile Phe Gly Pro Gly Leu Asn Ile Leu Ser Thr Tyr Ile Gly Ser Asp
            420                 425                 430

Thr Ala Thr Ala Thr Leu Ser Gly Thr Ser Met Ala Thr Pro His Val
        435                 440                 445

Val Gly Leu Leu Thr Tyr Phe Leu Ser Leu Gln Pro Asp Ala Asp Ser
    450                 455                 460

Glu Tyr Phe His Ala Ala Gly Gly Ile Thr Pro Ser Gln Leu Lys Lys
465                 470                 475                 480

Lys Leu Ile Asp Phe Ser Thr Lys Asn Val Leu Ser Asp Leu Pro Glu
                485                 490                 495

Asp Thr Val Asn Tyr Leu Ile Tyr Asn Gly Gly Gly Gln Asp Leu Asp
            500                 505                 510

Asp Leu Trp Gly Lys Asp Tyr Ser Ile Gly Lys Glu Pro Ser Ala Asn
        515                 520                 525

Pro Glu Phe Ser Leu Glu Ser Leu Ile Asn Ser Leu Asp Ser Lys Thr
    530                 535                 540

Asp Ala Ile Phe Asp Asp Val Arg Gln Leu Leu Asp Gln Phe Asn Ile
545                 550                 555                 560

Ile

<210>  3
<211>  894
<212>  DNA
<213>  Kluyveromyces lactis

<400> 3
agtttcaata cccaaaacag tgctccttgg ggtctagctc gtatttctca tcgtgaaaag 60

ttgaatttag gatctttcaa caagtacttg tatgatgatg acgctggtaa aggtgttact 120

gcttacgttg ttgacactgg tgtcaatgtt aaccataagg actttgatgg cagagctgtt 180

tggggtaaga ctattccaaa agatgatcca gatgtagatg gaaatggtca cggtacccac 240

tgtgctggta ccatcggttc ggttcattat ggtgttgcta agaatgctga tatagttgcc 300

gttaaggttt tgagatctaa tggttctggt accatgtctg atgttgttaa aggtgtcgaa 360

tatgttgccg aagcacacaa gaaagctgtt gaagaacaaa agaaagggtt caagggttca 420

actgctaaca tgtctttggg tggtggtaaa tctccagcct tggatttggc cgtcaacgcc 480

gctgttaagg caggtgttca ttttgctgtt gctgccggta atgagaacca agatgcttgt 540

aacacttcgc ctgccgcggc tgagaatgct atcacggttg gtgcctccac attaagtgat 600

gaaagagctt acttttccaa ttggggtaaa tgtgtcgaca tctttggtcc gggtttgaat 660

atcttatcta cctacattgg ttctgatact gctactgcta ccttgtctgg tacttctatg 720

gccactcctc atgttgtcgg tttgctaaca tatttcttgt ccttgcaacc agatgctgat 780

agtgaatatt tccatgccgc tggcggtatt actccttccc aactcaagaa gaagttaatt 840

gatttctcta ctaagaacgt attgtccgat ctacctgaag ataccgtgaa ctac 894

<210> 4
<211> 1686
<212> DNA
<213> Kluyveromyces lactis

<400> 4
atgaagttcg aaaatacatt attgactata accgcattgt ctaccgtggc tactgctttg 60

gttatccctg aagttaatag ggaaaacaag catggtgaca agagcgttgc catcaaagat 120

catgcttctt ctgatttgga taagcctcaa catcatgcta atggcaaggc tcgttctaag 180

tctcgtggtc gctgcgcaga ctccaagaaa ttcgacaagc tacgtccagt cgacgatgct 240

tcagctattt tagctccact ttctacagtt aatgatattg ccaacaagat tcctaatcgt 300

tacatcattg tctttaagaa agatgcctct gcagatgaag tgaagttcca tcaagaacta 360

gtctctgtcg aacatgccaa ggcactaggt tccttagctg acaatgaccc attcttcaca 420

gcaacttccg gtgaacatag tgaatttggt gtcaaagcac actctttgga aggtggtatt 480

caagactctt ttgatattgc cggttccctt tctggttatg ttggctactt cacaaaagaa 540

gttatcgatt tcatcagaag aagcccattg gttgaatttg ttgaagaaga ttctatggtt 600

ttctctaata gtttcaatac ccaaaacagt gctccttggg gtctagctcg tatttctcat 660

cgtgaaaagt tgaatttagg atctttcaac aagtacttgt atgatgatga cgctggtaaa 720

ggtgttactg cttacgttgt tgacactggt gtcaatgtta accataagga ctttgatggc 780

agagctgttt ggggtaagac tattccaaaa gatgatccag atgtagatgg aaatggtcac 840

ggtacccact gtgctggtac catcggttcg gttcattatg gtgttgctaa gaatgctgat 900

atagttgccg ttaaggtttt gagatctaat ggttctggta ccatgtctga tgttgttaaa 960

ggtgtcgaat atgttgccga agcacacaag aaagctgttg aagaacaaaa gaaagggttc 1020

aagggttcaa ctgctaacat gtctttgggt ggtggtaaat ctccagcctt ggatttggcc 1080

gtcaacgccg ctgttaaggc aggtgttcat tttgctgttg ctgccggtaa tgagaaccaa 1140

gatgcttgta acacttcgcc tgccgcggct gagaatgcta tcacggttgg tgcctccaca 1200

ttaagtgatg aaagagctta cttttccaat tggggtaaat gtgtcgacat ctttggtccg 1260

ggtttgaata tcttatctac ctacattggt tctgatactg ctactgctac cttgtctggt 1320

acttctatgg ccactcctca tgttgtcggt ttgctaacat atttcttgtc cttgcaacca 1380

gatgctgata gtgaatattt ccatgccgct ggcggtatta ctccttccca actcaagaag 1440

aagttaattg atttctctac taagaacgta ttgtccgatc tacctgaaga taccgtgaac 1500

tacttgattt acaacggtgg tggtcaagat ttggatgacc tatggggtaa ggattactct 1560

attggaaaag aaccatctgc caaccctgaa ttcagcttgg aaagcttgat taactctttg 1620

gattcaaaga ctgatgctat ctttgacgac gttagacagt tgttggacca atttaatatc 1680

atctaa 1686

<210> 5
<211> 40
<212> PRT
<213> Kluyveromyces lactis

<400> 5

Glu Lys Leu Asn Leu Gly Ser Phe Asn Lys Tyr Leu Tyr Asp Asp Asp
1               5                   10                  15

Ala Gly Lys Gly Val Thr Ala Tyr Val Val Asp Thr Gly Val Asn Val
            20                  25                  30

Asn His Lys Asp Phe Asp Gly Arg
        35                  40

<210> 6
<211> 8
<212> PRT
<213> Kluyveromyces lactis

<400> 6

Asn Ala Asp Ile Val Ala Val Lys
1               5

<210>  7
<211>  23
<212>  PRT
<213>  Kluyveromyces lactis

<400>  7

Ser Asn Gly Ser Gly Thr Met Ser Asp Val Val Lys Gly Val Glu Tyr
1               5                   10                  15

Val Ala Glu Ala His Lys Lys
            20

<210>  8
<211>  25
<212>  PRT
<213>  Kluyveromyces lactis

<400>  8

Gly Ser Thr Ala Asn Met Ser Leu Gly Gly Gly Lys Ser Pro Ala Leu
1               5                   10                  15

Asp Leu Ala Val Asn Ala Ala Val Lys
            20                  25

<210>  9
<211>  8
<212>  PRT
<213>  Kluyveromyces lactis

<400>  9

Ala Tyr Phe Ser Asn Trp Gly Lys
1               5

## Description

### Technical Field

The present invention relates to proteinase B, and a lactase solution and a method for producing the same using properties of proteinase B, and dairy products using the lactase solution, and the like.

### Background Art

Lactose intolerance is a condition, which shows various symptoms such as abdominal pains and diarrhea due to lactose in food such as dairy products because lactose cannot be congenitally decomposed. Lactose is a disaccharide formed from galactose and glucose. In order to deal with lactose intolerance, lactose contained in e.g. milk is decomposed into galactose and glucose beforehand with an enzyme lactase in the food manufacturing industry.

A lactase solution used to decompose lactose in e.g. milk has been conventionally produced by culturing a lactase-producing microorganism, extracting lactase from cells, removing foreign substances derived from cultures for purification, then adding a stabilizer and carrying out filtration sterilization.

Patent Literature 1 (JP 60-18394 B) discloses an invention relating to a method for producing lactase from cultures of a strain of *Kluyveromyces lactis.* According to this method, a crude enzyme solution obtained after autolysis of yeast bodies is applied to a DEAE cellulose column and eluted by salt concentration gradient to obtain two active fractions (lactase A and lactase B). It is also disclosed that in these two active fractions, various properties including temperature stability are almost same except that pH stability is slightly different, and an enzyme preparation can be formed from a mixture thereof. In addition, according to the gene analysis of lactase of *Kluyveromyces lactis,* this lactase is a polypeptide including 1025 amino acids and is estimated to have a molecular weight of 117,618 (Non Patent Literature 1).

It is further described that the lactase described in Patent Literature 1 has an optimum temperature of 40 to 50°C, and is deactivated by 45% at 50°C for 10 minutes and by 100% at 55°C for 10 minutes at pH 7.0.

### Citation List

### Patent Literature

Patent Literature 1: JP 60-18394 B
Patent Literature 2: JP 2004-534527 A
Patent Literature 3: JP 2009-517061 A

### Non Patent Literature

Non Patent Literature 1: Poch et al., Gene 1992 Sep 1; 118(1):55-63

### Summary of Invention

### Technical Problem

In practice, however, a phenomenon in which lactase products have different heat stability occurs, and it has been found that the previously reported stability of lactase is not necessarily applied to all products. Following this, there is often a problem in that stability during storage is different in lactase products. Accordingly, a factor to influence heat stability of lactase is to be present in lactase products; however, this factor is not described in these patent literatures at all.

Therefore, an object of the present invention is to identify a factor to influence heat stability of lactase in lactase products, and to provide a lactase solution with good heat stability as desired by using such factor as an index.

### Solution to Problem

Commercially available lactase products contain foreign proteins other than lactase. In order to remove these, it is common to carry out various purification treatments. When comparing long-term stability, however, highly purified lactase products have not necessarily had high stability.

When these commercially available lactase products are applied to SDS-PAGE, lactase has mainly a high molecular weight band (about 120 kDa). However, those in which the band of lactase is fragmented and several bands (80, 50, 33 and 32 kDa) are observed, are frequently found. It has been revealed that this lactase having several fragmented bands tends to have low heat stability.

Therefore, the present inventors succeeded in newly identifying proteinase B (which can be described as PrB hereinafter) contained in lactase products, which is a factor to fragment lactase. The present inventors found that a product in which lactase is not easily fragmented and which has high stability as desired could be produced by adjusting the amount and activity of such PrB, thereby completing the present invention.

Thus, the followings are provided by the present invention.
[1] Proteinase B having the following enzymatic properties,
   1) having an optimum temperature of about 40°C,
   2) having an optimum pH of about 8.0,
   3) being stable at pH 5.0 to 8.0,
   4) having high substrate specificity to FITC-casein and lactase,
   5) having a neutral lactase-fragmenting action, and
   6) having a molecular weight of about 29,700 to 30,000 (by SDS-PAGE);
[2] The proteinase B according to [1], satisfying the following (a) or (b),
   (a) a polypeptide whose amino acid sequence is represented by SEQ ID NO:1 in the sequence listing, and
   (b) a polypeptide derived from the polypeptide of (a) by deletion, substitution or addition of one or several amino acid residues in the amino acid sequence represented by SEQ ID NO:1 in the sequence listing, wherein its amino acid sequence has a homology of not less than 70% to the sequence of SEQ ID NO:1, and having a neutral lactase-fragmenting action at pH 5.0 to 8.0;
[3] A precursor of proteinase B, satisfying the following (a) or (b),
   (a) a polypeptide whose amino acid sequence is represented by SEQ ID NO:2 in the sequence listing, and
   (b) a polypeptide derived from the polypeptide of (a) by deletion, substitution or addition of one or several amino acid residues in the amino acid sequence represented by SEQ ID NO:2 in the sequence listing, wherein its amino acid sequence has a homology of not less than 70% to the sequence of SEQ ID NO:1, and its mature protein is a polypeptide having a neutral lactase-fragmenting action at pH 5.0 to 8.0;
[4] A polynucleotide coding for an amino acid sequence forming proteinase B according to [1] or [2], or the precursor of proteinase B according to [3];
[5] The polynucleotide according to [4], selected from the group consisting of the following (A) to (D),
   (A) a polynucleotide whose nucleotide sequence is represented by SEQ ID NO:3 in the sequence listing, or
   (B) a polynucleotide which hybridizes with the polynucleotide whose nucleotide sequence is represented by SEQ ID NO:3 in the sequence listing under stringent condition, and which codes for a polypeptide having a neutral lactase-fragmenting action at pH 5.0 to 8.0,
   (C) a polynucleotide whose nucleotide sequence is represented by SEQ ID NO:4 in the sequence listing, or
   (D) a polynucleotide which hybridizes with the polynucleotide whose nucleotide sequence is represented by SEQ ID NO:4 in the sequence listing under stringent condition, and which codes for a polypeptide including an amino acid sequence whose mature protein is a polypeptide having a neutral lactase-fragmenting action at pH 5.0 to 8.0;
[6] A lactase solution containing less than 11.5 ng of proteinase B according to [1] or [2] per unit of neutral lactase activity;
[7] The lactase solution according to [6], containing not less than 0.01 ng of proteinase B according to [1] or [2] per unit of neutral lactase activity;
[8] The lactase solution according to [6] or [7], which contains a protease inhibitor;
[9] The lactase solution according to any one of [6] to [8], which contains a stabilizer;
[10] The lactase solution according to any one of [6] to [9], for use in UHT milk or yogurt;
[11] A method for producing a lactase solution according to anyone of [6] to [10],
   the method including the steps of removing the proteinase B from the lactase solution and/or reducing the action of the proteinase B;
[12] The method for producing a lactase solution according to [11], wherein the step of removing the proteinase B is carried out by weakly basic anion-exchange column chromatography or hydrophobic interaction chromatography to the lactase solution;
[13] The method for producing a lactase solution according to [11], wherein the step of removing the proteinase B is carried out by treating the lactase solution with activated carbon;
[14] The method for producing a lactase solution according to anyone of [11] to [13], wherein the step of reducing the action of the proteinase B is carried out by treating the lactase solution with heat; and
[15] A method for evaluating the heat stability of lactase comprising measuring an amount of the proteinase B protein according to [1] or [2] and a lactase activity thereof, contained in a lactase solution, and using the amount of proteinase B protein per unit of lactase activity as an index.
[16] An antibody that binds specifically to the polypeptide whose amino acid sequence is represented by SEQ ID No.1.
[17] The antibody according to [16], which is a polyclonal antibody against the peptide whose amino acid sequence is a portion of the amino acid sequence represented by SEQ ID No.1, from positions 27 to position 45.

### Advantageous Effects of Invention

According to the present invention, there is provided proteinase B, as well as a lactase solution and a method for producing the same, using properties of proteinase B, in which the lactase protein is not easily fragmented and has good stability as desired,.

### Brief Description of Drawings

Fig. 1 is a sequence showing the deduced amino acid sequence of proteinase B of the present invention.
Fig. 2 is an image showing the state of fragmentation of the band of YNL A and YNL B by SDS-PAGE.
Fig. 3 is a graph showing the heat stability of YNL A and YNL B using the remaining activity of lactase.
Figs. 4 are images showing the results of SDS-PAGE obtained by adding PrB and a protease inhibitor to purified YNL (neutral lactase solution).
Figs. 5 are graphs showing heat stability obtained by adding PrB and a protease inhibitor to purified YNL (neutral lactase solution) using the remaining activity of lactase.
Fig. 6 is an image showing the state of fragmentation of lactase accompanied with the addition of PrB by SDS-PAGE to obtain the optimum value of the amount of PrB contained.
Fig. 7, Panel A is a graph showing the results of separation of PrB by column chromatography when using a resin (DEAE-Sepharose), and Panel B is a graph when using a resin (Butyl-Toyopearl).
Fig. 8 is a Western blotting image showing the effect of industrial resins removing PrB.
Fig. 9 is a Western blotting image showing the results of PrB detection in a liquid treated with activated carbon.
Fig. 10 is a Western blotting image showing the results of PrB detection before and after heat treatment.

### Description of Embodiments

The lactase solution involved in the present invention is a lactase solution having a small amount of proteinase B and/or a reduced lactase protein-fragmenting activity. The lactase solution may contain also a stabilizer and a protease inhibitor. The present invention will now be described in the order of (1) the properties of proteinase B, (2) the properties of a lactase solution, (3) a method for producing the lactase solution, and (4) the uses of the lactase solution.

### «Properties of Proteinase B (PrB)»

The proteinase B (PrB), which is contained in the lactase solution of the present invention and expected to be an enzyme having the lactase-fragmenting activity, will now be described in detail. It is expected that by removing this proteinase B from the lactase solution and/or suppressing the activity, the fragmentation of lactase can be suppressed. It should be noted that proteinase B contained in lactase was identified for the first time in the present invention, and has not been able to be detected by a conventional technique, a measurement method using casein as a substrate.

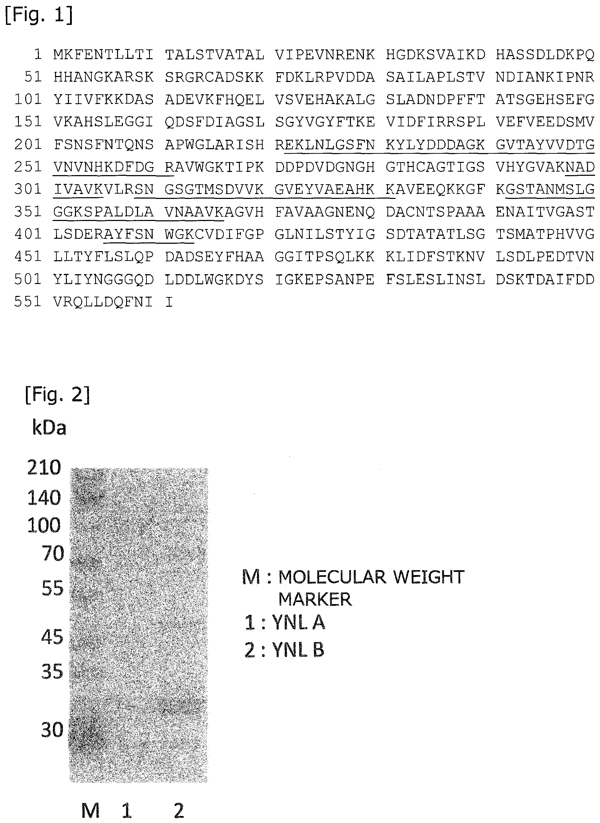

The amino acid sequence of a protein having the lactase-fragmenting activity, purified from cell extract of *Kluyveromyces lactis* was analyzed by LC-MS/MS, and was subjected to Mascot search. The underlined parts of the amino acid sequence (SEQ ID NO:2) shown in Fig. 1 are parts identified by the LC-MS/MS analysis, and the others are parts estimated by Mascot search. Furthermore, the amino acid sequence was analyzed about a homology to existing protein sequences in databases using BLAST, and it was consequently matched to a protein with unknown function of *Kluyveromyces lactis,* while it was confirmed to have a homology of about 68% to PrB derived from *S.cerevisiae.* The amino acid sequence was also 100% matched to an amino acid sequence translated from the base sequence information of such protein gene cloned from *Kluyveromyces lactis* genome. Thus, it was concluded that such protein having the lactase-fragmenting activity is PrB derived from *Kluyveromyces lactis.*

We revealed the properties shown in Table 1 using purified PrB of the present invention. This enzyme is believed to be a neutral protease as with PrB derived from *S.cerevisiae.* As shown in Table 2, the different rate of reaction to Azocoll and higher substrate specificity to lactase of the enzyme showed that the substrate specificity of the enzyme were different from those of PrB derived from S.cerevisiae.

Besides, the enzyme has various enzymological properties and substrate specificity as shown in Tables 1 and 2 below. The enzyme has an optimum temperature of about 40°C and an optimum pH of about 8.0, and is stable at pH 5.0 to 8.0. The enzyme also has high substrate specificity to FITC-casein and lactase, and the molecular weight thereof is about 29,700 to 30,000 (by SDS-PAGE).

### [Table 1]

**[Table 1]**

| VARIOUS ENZYMOLOGICAL PROPERTIES | | | |
|---|---|---|---|
| | *K. lactis* | | *S.cerevisiae* |
| | FITC-CASEIN-DECOMPOSING ACTIVITY | LACTASE-FRAGMENTING ACTIVITY | AZOCOLL-DECOMPOSING ACTIVITY |
| MOLECULAR WEIGHT (kDa) | 30 | - | 33 |
| OPTIMUM REACTION pH | pH 8.0 | pH 7.0 | pH 7.0 |
| OPTIMUM REACTION TEMPERATURE | 40°C | 40°C | - |
| pH STABILITY | pH 5.0∼8.0 | - | - |
| HEAT STABILITY | 40°C | - | 25°C |
| ACTIVATOR | Ca²⁺ | - | - |
| INHIBITOR | Cu²⁺, Fe²⁺ | AEBSF | PCMB, PMSF, HgCl₂ |

| | | | |
|---|---|---|---|
| - : NOT MEASURED | | | |

### [Table 2]

**[Table 2]**

| SUBSTRATE SPECIFICITY | | |
|---|---|---|
| SUBSTRATE | *K.lactis* | *S. cerevisiae* |
| Azocasein | 1 | 1 |
| Azocoll | 1.9 | 9.1 |
| FITC-casein | 78 | - |
| Bz-Tyr-pNa | 0 | 0.2 |
| Bz-Arg-pNa | 0 | - |
| Ac-Phe-pNa | 0 | - |
| Lactase | 1159 | - |
| BSA | 0 | - |
| Casein | 0 | - |

| | | |
|---|---|---|
| - : NOT MEASURED | | |

The neutral protease genes generally have a long prepro-sequence. The pre-sequence is required to transport a protein, while the pro-sequence is an unnecessary sequence when the activated conformation of an enzyme is formed. The present inventors found the full gene sequence coding for a neutral protease precursor containing the prepro-sequence shown in SEQ ID NO:4 in the sequence listing, and found the amino acid sequence of the precursor shown in SEQ ID NO:2. Finally, from this neutral protease precursor, the present inventors found the amino acid sequence of the neutral protease of the present invention, shown in SEQ ID NO:1, i.e. the amino acid sequence of the mature enzyme, that is produced outside the cells, and the gene sequence coding for this, shown in SEQ ID NO:3.

The proteinase B derived from *Kluyveromyces lactis* which is preferred for the present invention, is a polypeptide having the amino acid sequence forming the mature enzyme, shown in SEQ ID NO:1, or the same amino acid sequence except that one or several amino acids are deleted, substituted or added, wherein the amino acid sequence has a homology of not less than 70% to such sequence, and more preferably a polypeptide having the amino acid sequence forming the mature enzyme, shown in SEQ ID NO:1, or the same amino acid sequence except that one or several amino acids are deleted, substituted, inverted, added or inserted.

Another preferred aspect of proteinase B of the present invention is a polypeptide having the amino acid sequence forming a precursor of proteinase B, shown in SEQ ID NO:2, or the same amino acid sequence except that one or several amino acids are deleted, substituted or added, wherein the amino acid sequence has a homology of not less than 70% to such sequence, and more preferably a polypeptide having the amino acid sequence forming the mature enzyme, shown in SEQ ID NO:1, or the same amino acid sequence except that one or several amino acids are deleted, substituted, or added. This precursor functions as a mature protein with a molecular weight of about 30,000 Da through the maturation process due to the properties as a protease.

The genes including a gene coding for an amino acid sequence of the neutral protease of the present invention or a precursor thereof are nucleotide sequences corresponding to this.

The gene coding for an amino acid sequence of preferred proteinase B of the present invention or a precursor thereof is specifically a polynucleotide selected from the group consisting of the following (A) to (D):
(A) a polynucleotide including the nucleotide sequence shown in SEQ ID NO:3 in the sequence listing, or
(B) a polynucleotide which hybridizes with the polynucleotide including the nucleotide sequence shown in SEQ ID NO:3 in the sequence listing under stringent conditions, and which codes for the polypeptide having a neutral lactase-fragmenting action at pH 5.0 to 8.0,
(C) a polynucleotide including the nucleotide sequence shown in SEQ ID NO:4 in the sequence listing, or
(D) a polynucleotide which hybridizes with the polynucleotide including the nucleotide sequence shown in SEQ ID NO:4 in the sequence listing under stringent conditions, and which codes for the polypeptide including an amino acid sequence whose mature protein is a polypeptide having a neutral lactase-fragmenting action at pH 5.0 to 8.0.

With respect to the amino acid sequence of a peptide, the "polypeptide including an amino acid sequence except that one or several amino acid residues are deleted, substituted or added" used in the description means a variant of the polypeptide including the amino acid sequence shown in a sequence number, which has a lactase-fragmenting action with a degree equal or similar to that of the polypeptide including the amino acid sequence shown in the sequence number (e.g. not less than 50%, preferably not less than 80%, and more preferably not less than 100%). Such variant polypeptide can be also a polypeptide including an amino acid sequence having a sequence homology of not less than 70%, preferably not less than 80%, and further preferably not less than 90% to the amino acid sequence shown in the sequence number.

In addition, the "stringent conditions" in the description include conditions described in for example "Molecular Cloning: A Laboratory Manual 2nd ed." (T. Maniatis et al., published by Cold Spring Harbor Laboratory, 1989) and the like, and include more specifically a condition that hybridization is carried out by storage with a probe at a temperature 50 to 65°C for one night in a solution including for example 6 x SSC (the composition of 1 x SSC: 0.15 M NaCl, 0.015 M sodium citrate, pH 7.0), 0.5% SDS, 5 x Denhardt, and 100 µg/mL heat-denatured herring sperm DNA, and the like.

The sequence homology in the description refers to, when two amino acid sequences are aligned by introducing gaps or without introducing gaps so that the sequence identity will be greatest, the percentage (%) of the number of identical amino acid residues to the total number of amino acids including gaps. The sequence homology % can be determined using known algorithms such as BLAST and FASTA released by NCBI, USA.

The proteinase B of the present invention includes a fragment of the proteinase B retaining a neutral lactase-fragmenting action. The fragment may have any length as long as it retaines the ability to fragment neutral lactase. The fragment length is preferably a length of at least 20 amino acid residues, more preferably at least 25 residues, e.g. 30, 35 or 40 residues, or more.

In addition, the "lactase-fragmenting action" in the claims and description can be an action by which the neutral lactase around 120 kDa in SDS-PAGE is fragmented, and preferably the fragments of the fragmented lactase show the neutral lactase activity.

The PrB of the present invention has the above-described enzymological properties as a mature protein, and preferably includes a polypeptide including an amino acid sequence selected from the amino acid sequences (i) to (v) shown below, or having an amino acid sequence of the amino acid sequences (i) to (v) shown below except that one or several amino acid residues are deleted, substituted or added. The PrB of the present invention is a polypeptide more preferably including two or more amino acid sequences of the amino acid sequences (i) to (v) mentioned below, or these amino acid sequences except that one or several amino acid residues are deleted, substituted or added, further preferably including three or more amino acid sequences or these amino acid sequences except that one or several amino acid residues are deleted, substituted or added, particularly preferably including 4 or more amino acid sequences or these amino acid sequences except that one or several amino acid residues are deleted, substituted or added, and most preferably including all the amino acid sequences mentioned below or these amino acid sequences except that one or several amino acid residues are deleted, substituted or added. Preferably, among the above, the PrB of the present invention is one including a larger number of amino acid residues of the amino acid sequences (i) to (v) mentioned below, or includes them in descending order of amino acid residue:
(i) EKLNLGSFNKYLYDDDAGKGVTAYVVDTGVNVNHKDFDGR (SEQ ID NO:5),
(ii) NADIVAVK (SEQ ID NO:6),
(iii) SNGSGTMSDVVKGVEYVAEAHKK (SEQ ID NO:7),
(iv) GSTANMSLGGGKSPALDLAVNAAVK (SEQ ID NO:8), and
(v) AYFSNWGK (SEQ ID NO:9).

### (Method for Purifying PrB)

The method for obtaining PrB of the present invention is not particularly limited, and, for example, PrB can be purified from microorganism extract. More specifically, the supernatant obtained by centrifugal separation after crushing cells with ultrasonic treatment is applied as a crude enzyme solution to DEAE-Sepharose (manufactured by GE Healthcare) column chromatography. The obtained active fraction can be collected, and then applied to Butyl-Toyopearl (manufactured by Tosoh Corporation) column chromatography, and the active fraction obtained by elution with linear gradient can be collected. It should be noted that the active fraction obtained by hydroxyapatite column chromatography is confirmed to be electrophoretically single.

It is believed that because the PrB of the present invention has a lactase protein-fragmenting activity as described above, when contained in a lactase solution, the heat stability thereof is lost, but using its characteristic substrate specificity, PrB can be also used for various uses. The uses for modifying the physical properties of e.g. low-allergy food, seasonings, dairy products are for example thought.

### «Properties of Lactase Solution»

The properties of the lactase solution of the present invention will now be described in detail.

### <Origin of Fungal Species for Lactase>

The lactases used in the present invention are lactases derived from yeasts (*Kluyveromyces* genus). Almost all of them are so-called neutral lactases with an optimum pH of pH 6 to pH 8. Examples of lactase producing yeasts among Kluyveromyces genus include *Kluyveromyces lactis, Kluyveromyces fragillis, Kluyveromyces marxianus* and the like.

### <Activity of Lactase Solution>

The lactase solution of the present invention desirably has a lactase activity of 10 to 100,000 NLU/g. The "NLU" means Neutral Lactase Unit. The method for measuring activity is as follows. The NLU is measured using the hydrolysis of a substrate, o-nitrophenyl-β-galactopyranoside (ONPG), into o-nitrophenol and galactose. The reaction is finished by adding sodium carbonate. The color of the formed o-nitrophenol turns into yellow in an alkaline medium and changes in absorbance are used to measure enzyme activity (represented by NLU/g). This procedure is published in Lactase (neutral) (β-galactosidase) activity on pages 801 to 802 in Food Chemicals Codex (FCC) 4th Edition, July 1, 1996.

### <Heat Stability of Lactase and Fragmentation Thereof>

Herein, it was found that when the band by SDS-PAGE is fragmented as described below, the heat stability of lactase tended to be low. It can be said that the lactase solution of the present invention is one in which the fragmentation of lactase is suppressed by reducing the fragmentation activity of PrB to improve heat stability.

### (Confirmation of Fragmentation)

First, the state of fragmentation of lactase in a lactase solution can be confirmed by SDS-PAGE using 10% polyacrylamide gel. For example, a lactase solution is diluted with purified water as needed, and mixed with Sample Buffer for SDS-PAGE at 1 : 1, and electrophoresis samples are prepared by heat treatment at 100°C for 3 minutes. Next, the standard and electrophoresis samples are applied to 10% acrylamide gel, and subjected to electrophoresis. The molecular weight of lactase can be also roughly estimated by comparison with the standard. As the standard, CLEARLY Stained Protein Ladder (Takara #3454A) and the like are used. The gel after electrophoresis is subjected to protein staining using a CBB staining solution (BIO-RAD #161-0786).

The gel after staining was scanned as a grayscale image with a scanner GT-X820 manufactured by Seiko Epson Corporation. Furthermore, the concentration of each band (protein amount) can be also measured by Image J software (NIH, Bethesda, MD). The fragmentation of lactase can be considered in more detail by analyzing the concentration of each band with the software.

### <Amount of PrB in Lactase Solution>

In the lactase solution of the present invention, the amount of PrB contained is preferably small. The fragmentation of lactase is believed to reduce the heat stability of lactase eventually, and thus PrB is believed to contribute to the fragmentation of lactase. That is to say, as the amount of PrB contained decreases, the heat stability of a lactase solution tends to increase. As the amount in the lactase solution of the present invention, less than 11.5 ng of proteinase B of the present invention is preferably contained per unit of neutral lactase activity (1 NLU) in terms of improvement in heat stability, more preferably not more than 3.0 ng, and further preferably not more than 0.30 ng.

The lower limit of the amount of PrB contained in the lactase solution of the present invention is not particularly limited, and is 0.01 ng/NLU. The effect on the stability of lactase hardly improves even when the amount is less than 0.01 ng/NLU, and thus decreasing less than 0.01 ng/NLU is not economical.

### (Determination of PrB by Western blot)

PrB can be confirmed by Western blotting. A PrB solution purified by the above-mentioned method was 10-fold diluted using x 1 SDS-PAGE Sample buffer, and was further subjected to two-fold serial dilution. Each diluted solution was treated with heat at 100°C for 3 minutes to prepare electrophoresis samples. A lactase product was diluted with x 1 SDS-PAGE Sample buffer so that the concentration was 200 NLU/mL to prepare electrophoresis samples in the same manner. To SDS-PAGE, 10 µL of each electrophoresis sample was applied, and was subjected to electrophoresis at a constant current of 20 mA. One of the gel after electrophoresis was subjected to protein staining using a CBB staining solution in the same manner as above, and another one was applied to Western blot. As the transfer membrane for Western blot, Immun-Blot PVDF Membrane For Protein Blotting (BIO-RAD #162-0174) was used, and the protein was transferred by a semi-dry type. The membrane after transfer was blocked with a 6% skim milk solution, and then washed with Tween-PBS.

As the primary antibody, an anti-PrB polyclonal antibody was prepared by immunizing rabbit with a synthetic peptide (CNKYLYDDDAGKGVTAYVVD) as an antigen. As the second antibody, Goat anti-rabbit IgG (H + L) Horseradish Peroxidase Conjugate (BIO-RAD #170-6515) was used. After washing with Tween-PBS, detection was carried out by a chemiluminescent method using Chemi-Lumi One Super (nacalai tesque #02230-30). The membrane was exposed to an X-ray film for 5 minutes, which was then developed. The film after development was scanned as a grayscale image with a scanner GT-280 manufactured by Seiko Epson Corporation, and the concentration of each band (protein amount) was measured by Image J software (NIH, Bethesda, MD). For a quantitative analysis, a software attached to iMark Microplate reader (BIO-RAD) was used. The band area of PrB was plotted along the ordinate and the amount of PrB protein was plotted along the abscissa to obtain a regression equation y = -0.366/(1 + (x/32.3)^{3.474}) + 0.366. Therefore, the amount of protein per lane was calculated by substituting the band area of a sample for y in the equation.

Thus, in one embodiment of the present invention, there is provided an antibody that binds specifically to PrB. The term "specifically binding" of an antibody means that the antibody binds to a target substance, but does not bind to other substances. Whether or not an antibody binds to a target substance, and/or does not bind to other substances, may be confirmed by any methods that detect antigen-antibody reactions such as southern hybridization, PCR, western blotting, and ELISA. The phrase "does not bind to" means that the binding of the non-binding protein or peptide is lower than that of the target substance in a sufficiently distinguishable manner. For example, comparing to PrB of the present invention, a cross-reactivity to PrB derived from *S. cervisiae* may be lower than 1%, lower than 0.5%, lower than 0.3%, lower than 0.1%, lower than 0.05% and lower than 0.03%.

In a preferable embodiment, the above-prepared primary antibody is a polyclonal antibody against the peptide whose amino acid sequence is a portion of the amino acid sequence represented by SEQ ID No.1, from position 27 to position 45. The polyclonal antibody, as described in the following examples, is capable of detecting residual PrB efficiently in each purification step of lactase.

### (Evaluation of PrB Using Index of Fragmentation Activity)

As a method for evaluating an influence on the fragmentation activity of PrB, the "lactase-fragmenting activity" can be also used as its index. This "lactase-fragmenting activity" indicates the enzyme activity of fragmenting the lactase protein, and an actual index can be defined as follows. When the band strength around 120 kDa of a sample to which PrB is not added is regarded as 100%, the band area relative value around 120 kDa of samples obtained by adding 1 to 4 mACU of PrB is found as a relative value (1 ACU means the amount of enzyme which raises the absorbance at 428 nm by 1 at 30°C for an hour using azocasein as a substrate). Next, the amount of enzyme added when the lactase band after reaction at 30°C for an hour decreases by 20% compared to when PrB is not added is regarded as 1 LDU (Lactase Degradation Unit) of lactase-fragmenting activity.

It should be noted that proteinase B contained in lactase and/or the amount of protein in a fragment thereof and lactase activity are measured, and the heat stability of lactase can be also evaluated using proteinase B per unit of lactase activity and/or the amount of protein in the fragment thereof as an index.

### (Confirmation of Heat Stability)

About a lactase solution before and after heat treatment, the activity of the lactase solution is measured in the above-described method, and heat stability can be confirmed by comparing the activity before and after heat treatment. About the effect of each treatment step, for example an untreated sample and a post-treatment sample are allowed to react under the same condition, and the effect can be confirmed by comparing the remaining activity.

### «Method for Producing Lactase Solution»

The lactase solution of the present invention can be one which is collected from a microorganism and purified and in which PrB activity is adjusted in the following method.

One example of the method for producing the lactase solution of the present invention includes the following 5 steps:
(1) the step of culturing a microorganism,
(2) the step of collecting lactase from the microorganism,
(3) the step of purifying the lactase,
(4) the step of adjusting the lactase-fragmenting activity of PrB, and
(5) the step of adjusting the lactase activity.

The details of the above-mentioned 5 steps will now be described. It should be noted however that the present invention is not limited to these methods as long as the effect of the present invention is produced.

### (1) Step of Culturing Microorganism

For the step of culturing a microorganism, a known medium is used, and a known strain can be used. The culture conditions are also known, and can be appropriately selected as needed.

### (2) Step of Collecting Lactase from Microorganism

In the case of intracellular enzyme, the step of collecting lactase from a microorganism is required to include the step of extracting lactase. This extraction step is not particularly limited as long as the step is a method by which lactase can be transferred outside cells, and a known extraction method can be used. On the other hand, in the case of lactase which is an enzyme secreted outside cells by e.g. gene introduction and variation, lactase is contained in the culture fluid, and thus the extraction step is not required.

### (3) Step of Purifying Lactase

The step of purifying lactase is important to obtain the lactase solution of the present invention. Patent Literature 1, Patent Literature 2 and Patent Literature 3 are common in the purification of the lactase solution by chromatography. By this chromatography, the purification of lactase proceeds and the lactase activity can be improved thereby. It turned out however that when lactase is purified using chromatography (e.g. partition chromatography or molecular sieve chromatography, adsorption chromatography or ion-exchange chromatography), there is the possibility that lactase, originally 120 kDa, can be decomposed to lactases with 80 kDa and 50 kDa when using some techniques. Although lactases with both molecular weights have the lactase activity, when the proportion of, particularly, a fraction with not more than 80 kDa increases by decomposition of lactase, the heat stability of lactase is reduced. Accordingly, there is the possibility to cause a problem in that lactose is difficult to decompose at a relatively high temperature.

The lactase of the present invention can be also obtained using salting-out and demineralization treatment in the purification step. That is, lactase is precipitated by salting-out, and the precipitates are then collected and redissolved to remove salts contained in the precipitates. The salting-out, and collection, redissolution and demineralization of precipitates may be successively carried out. As long as the lactase of the present invention can be obtained, other purification means can be also used in combination.

Salting-out agents for salting-out include ammonium sulfate, sodium sulfate, potassium phosphate, magnesium sulfate, sodium citrate, sodium chloride, and potassium chloride, and one or two or more of these agents can be used.

When ammonium sulfate is added as a salting-out agent to a solution containing lactase, 10 to 90% saturation is preferred and 30 to 70% saturation is further preferred. When other salting-out agents are used, an amount corresponding to such amount of ammonium sulfate added can be used.

Lactase can be precipitated from a solution containing lactase by adding a salting-out agent such as ammonium sulfate. As the conditions that lactase is precipitated by adding a salting-out agent, the solution is preferably left to stand at 1 to 40°C for 1 to 80 hours. The pH condition is preferably 4 to 9 at this time. Further preferably, the conditions are 4 to 25°C (room temperature), 1 to 48 hours, and pH 5 to 8. It should be noted that the lower limit of temperature condition can be a temperature at which a solution containing lactase is not solidified. After a solution which had contained lactase and precipitates containing lactase are subjected to solid-liquid separation by filtration, solid lactase is redissolved in e.g. water and a buffer solution, and demineralization treatment is carried out by dialysis and concentration with ultrafiltration.

### (4) Step of Adjusting of Lactase-Fragmenting activity of PrB

It is preferred that the method for producing the lactase of the present invention separately include the step of adjusting the amount of PrB and/or the activity thereof to improve its heat stability. However, when the fragmentation activity (action) of PrB can be sufficiently reduced by the above-mentioned purification step, the above-mentioned purification step can double as such adjustment step. These steps can be also applied to a finished product, a lactase solution. Several steps can be combined simultaneously. Each of the specific steps will now be described.

In these adjustment steps described below, preferably the lactase activity is not reduced before and after a step. For example, when the lactase activity before a step is regarded as 100%, the remaining activity of lactase after such step is preferably not less than 40%, and more preferably not less than 80%.

### (Step of Heat Treatment)

It is estimated that PrB is easily decomposed or deactivated with heat treatment. By heat treatment under the conditions that the lactase activity is not lost, and the conditions of heat treatment that the fragmentation activity of PrB can be reduced, the lactase solution of the present invention can be obtained. Such heat treatment conditions are preferably at 35 to 60°C for 10 or more to less than 180 minutes, and more preferably at 35 to 50°C for 30 minutes or more to 150 minutes or less.

### (Step of Adding Protease Inhibitor)

The method for producing the lactase solution of the present invention preferably includes the step of adding a protease inhibitor. The PrB activity can be suppressed by adding a protease inhibitor having the action of inhibiting PrB to a lactase solution, and consequently the lactase-fragmenting activity can be suppressed, and a lactase solution with higher heat stability can be obtained.

The type of protease inhibitor which can be used in the present invention is not particularly limited, and examples thereof include serine protease inhibitors and SH modifying reagents. The serine protease inhibitors include, as inhibitors for the sulfonylation of active centers, PMSF (phenylmethylsulfonyl fluoride), AEBSF (aminoethyl benzylsulfonyl fluoride), and, as inhibitors for alkylation, TLCK (tosyl lysine chloromethylketone), and TPCK (tosyl phenylalanine chlorometylketone). Other serine protease inhibitors are benzamidine, and peptide compounds such as aprotinin and ovomucoid. The SH modifying reagents include PCMB (p-chloromercuribenzoate), p-hydroxymercuribenzoate, and HgCl₂. A commercially available protease inhibitor cocktail (e.g. protease inhibitor cocktail #P8340-1 ML manufactured by SIGMA-ALDRICH) can be also used. Furthermore, these protease inhibitors can be used alone or two or more inhibitors can be mixed and used.

The amount of protease inhibitor added is not limited as long as the effect of the present invention is produced, and the amount added is preferably for example 0.1 to 1000 mM for AEBSF, 0.8 to 800 µM for aprotinin.

### (Step of Activated Carbon Treatment)

The method for producing the lactase solution of the present invention preferably includes the step of activated carbon treatment. By the activated carbon treatment, PrB can be removed, and consequently the lactase-fragmenting activity is suppressed, and a lactase solution with higher heat stability can be obtained. It should be noted that "removing PrB" in the description encompasses reducing the amount of PrB protein in lactase as described above to a preferred range or a range acceptable as a product.

The activated carbon treatment is not limited as long as the effect of the present invention is produced. For example, the step can be the step of obtaining a liquid treated with activated carbon by adding activated carbon at any timing in the process of the step of producing lactase solution (neutral lactase solution). The activated carbon to be used is not particularly limited, and the treatment is preferably carried out using TAIKO (manufactured by Futamura Chemical Co., Ltd.), Fuji Activated Carbon (manufactured by SERACHEM Co., Ltd.), SHIRASAGI (manufactured by Osaka Gas Chemicals Co., Ltd.), Hokuetsu (manufactured by Ajinomoto Fine-Techno Co., Inc.) and the like. These activated carbons can be used alone or can be also used as a liquid treated with activated carbon by mixing two or more activated carbons.

### (Step of Treatment with Chromatography)

As described above, treatment with chromatography can be used, in which PrB can be removed without causing the fragmentation of lactase (or the breakage of molecular chains). Examples of such treatment can include weakly basic anion-exchange column chromatography having diethylaminoethyl (DEAE) group, hydrophobic interaction chromatography having e.g. butyl group and phenyl group, and gel permeation chromatography. With respect to base materials for chromatography, those which are commonly used can be used.

### (Step of Treatment with Resin)

The method for producing the lactase solution of the present invention preferably includes the step of adsorbing PrB by mixing each resin with any lactase solution between the collection of lactase from a microorganism and the purification process. As the mixing method, a known method can be used. The resin is not limited as long as the effect of the present invention is produced, and examples thereof include IRA96SB, IRA904CL, HPA25L, FPL3500, XAD1180N, XAD7HP and the like.

In these methods for removing PrB, the removal of PrB and simultaneously the fragmentation of lactase can proceed. It is believed that this is because, for example, among the treatments with activated carbon and a resin, in a batch type in which a lactase solution and the resin are mixed to adsorb PrB, PrB adsorbed on the surface of activated carbon and a resin does not lose activity even after adsorption and contributes to the progression of the fragmentation of lactase. Therefore, it is believed that, in a method in which, for example, a reactive group such as an ion-exchange group is immobilized on a membrane and a lactase solution is transported across the membrane to adsorb PrB, the contact time of the lactase liquid and adsorbed PrB is slight, and the fragmentation of lactase can be suppressed. In terms of the contact time of PrB and a lactase solution, it is believed that the ion-exchange membrane treatment is also preferred for the present invention.

### (5) Step of Adjusting Lactase Activity

The step of adjusting lactase activity is not limited as long as the activity of lactase can be adjusted. Examples are the addition of an aqueous solution containing water and a salt, and the addition of a stabilizer, and the like.

### (Addition of Stabilizer)

The method for producing the lactase solution of the present invention preferably includes, as needed, the step of adding a stabilizer contributing to the stabilization of lactase activity. The lactase solution of the present invention can contain various components like this. The stabilizer is not particularly limited, and examples thereof can include metal salts, various saccharides, ascorbic acid, glycerin and the like which contribute to the stabilization of lactase, starch and dextrin which are filler to improve the ease of use, inorganic salts having a buffer action and the like. Among these, glycerin is more preferred because of not only a stabilizing effect but also a bacteriostatic effect.

The amount of stabilizer added is not limited as long as the effect of the present invention is produced, and is, for example, 10 to 50 mass% on the basis of a lactase solution.

### (Selection of Production Method and Cost Effectiveness)

In the present invention, a lactase solution with good heat stability can be obtained by adjusting the fragmentation activity of PrB as described above. However, high heat stability is not required depending on e.g. the uses of a lactase solution, and cost-effectiveness can be lowered by unnecessary steps, e.g. purification. In such case, high cost-effectiveness can be also produced by using the protein amount and/or fragmentation activity (LDU) of newly identified PrB as an index, and appropriately selecting the method for producing a lactase solution. Therefore, the method for removing PrB contained in a lactase solution (or reducing the action) and conditions and the like are determined based on the uses and the like depending on required stability, and cost-effectiveness can be maximized without purification (adjustment) beyond the level required thereby.

More specifically, first, the amount of PrB contained in a lactase extraction liquid is measured (as needed, in each stage of the production method). The purification method for obtaining a lactase solution of the present invention, the method for adjusting (reducing) the amount of PrB as the method for adjusting the lactase-fragmenting activity and condition setting, and the method for reducing PrB activity and condition setting, and the like are selected depending on a degree of heat stability required for desired uses (e.g. long-life milk), and the production method can be optimized thereby.

### «Uses of Lactase Solution»

Milk for raw materials is a target to which a lactase solution is added. In the present invention, known source milk can be used. The source milk includes one before sterilization and one after sterilization. The source milk is only required to be one using milk. Ingredients forming source milk include water, raw milk, sterilized milk, skim milk, whole milk powder, powdered skim milk, buttermilk, butter, cream, whey protein concentrate (WPC), whey protein isolate (WPI), α (alpha)-La, β (beta)-Lg and the like.

By adding the lactase solution of the present invention to source milk, lactose contained in such source milk can be decomposed. The decomposition temperature is 1 to 60°C, and the decomposition time is 10 minutes to 168 hours.

As the specific form of use, the lactase solution is used for e.g. producing dairy products. The methods for producing dairy products in which lactose is decomposed include 1. a method in which lactase is added to milk before sterilization to decompose lactose, and lactase is then deactivated simultaneously with the heat sterilization of milk (JP 5-501197 A), 2. a method in which lactase is added to sterilized milk to decompose lactose, and dairy products are then produced after lactase is deactivated by heat treatment, 3. a method in which by adding lactase to sterilized milk to decompose lactose at the distribution stage, heat treatment is not carried out and dairy products are produced without deactivating lactase, 4. a method in which lactose in milk is decomposed with immobilized lactase and dairy products are then produced (JP 46-105593 A, JP 59-162833 A), and 5. a method in which dairy products are produced using a raw ingredient, in which lactose is decomposed or lactose is removed in advance, for sterilized milk, and the like.

The lactase solution involved in the present invention is particularly suitable for producing dairy products. Here, dairy products mean, for example, milks such as ice cream and long-life milk, yogurt, fresh cream, sour cream, and cheese. In particular, the lactase solution involved in the present invention can be preferably used when applying heat load with 40°C or higher. Particularly, a lactase solution more suitable for heat load is easily obtained by adjusting the amount and/or activity of proteinase B of the present invention newly identified. Examples of such uses include milks, yogurt or UHT milk (long-life milk).

### Examples

The present invention will now be described in more detail by way of examples thereof. It should be noted however that the present invention is not limited to these examples.

### 1. Relationship between Fragmentation and Heat Stability of Lactase

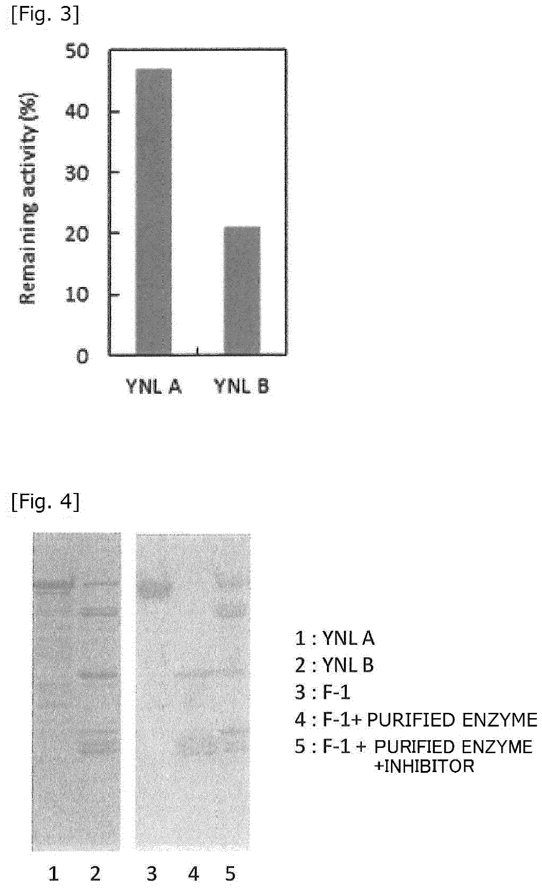

As prior consideration, the following test was carried out about a relationship between the fragmentation and heat stability of lactase. Using a commercially available lactase solution, GODO-YNL2 (manufactured by GODO SHUSEI CO., LTD., 5,000 NLU/g of neutral lactase activity), the fragmentation of lactase was confirmed by SDS-PAGE, and the heat stability test was carried out by measuring the remaining activity of lactase. It should be noted that two GODO-YNL2 products with different lots (YNL A, YNL B) were tested. As the conditions of the heat stability test, using an enzyme solution (lactase solution) diluted to 0.25 NLU/g, the activity after treatment at 50°C for 10 minutes was calculated as the remaining activity (%) to that of an untreated sample. The results of SDS-PAGE were shown in Fig. 2 and the results of the heat stability test were shown in Fig. 3. From these results, it is found that YNL B in which lactase is more fragmented has lower heat stability. Accordingly, a relationship between the fragmentation and heat stability of lactase was suggested.

### 2. Confirmation of Fragmentation of Lactase by Addition of Purified PrB and Suppression of Fragmentation of Lactase by Addition of Protease Inhibitor

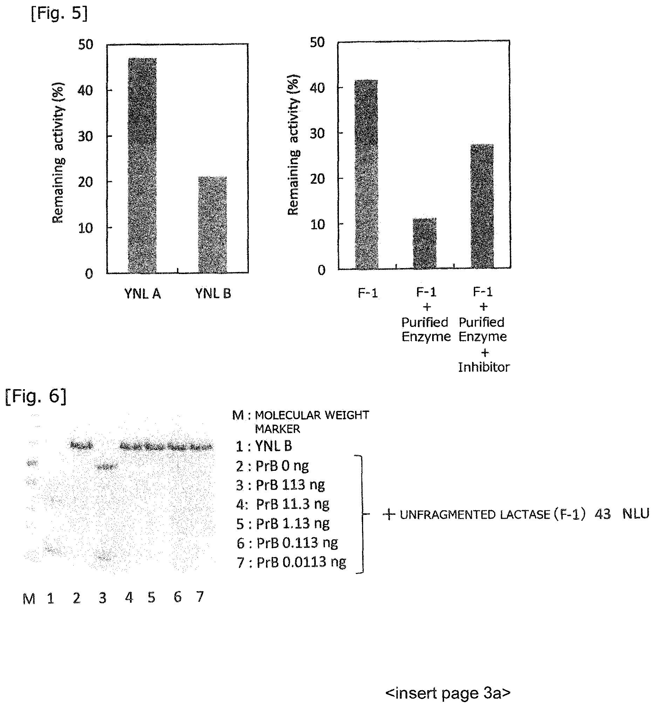

YNL A was applied to gel permeation chromatography (HiPrep Sephacryl S-200 High Resolution manufactured by GE Healthcare) to prepare a lactase substrate containing only a high molecular fraction and not being fragmented (F-1). A sample obtained by adding purified PrB to this unfragmented lactase (F-1) to react (F-1 + purified enzyme), and a sample obtained by adding a protease inhibitor cocktail (SIGMA-ALDRICH #P8340-1 ML) thereto (F-1 + purified enzyme + inhibitor) were prepared. These were subjected to SDS-PAGE and the heat stability test (the conditions are the same as above). The results of SDS-PAGE and the results of the heat stability test are shown in Fig. 4 and Figs. 5, respectively. From the results in Fig. 4, it was found that the fragmentation of lactase proceeded by addition of PrB and this fragmentation was inhibited by a protease inhibitor. From the results in Figs. 5, as a sample was fragmented, its heat stability tended to be lowered as described above. Accordingly, a relationship between the fragmentation of lactase by purified PrB and heat stability attributed to fragmentation could be shown.

### 3. Consideration of Amount of PrB not Causing Fragmentation in YNL (neutral lactase solution)

The fragmentation of lactase is estimated to depend on the amount of PrB in lactase preparation. Therefore, the unit of PrB at which lactase is not fragmented even when PrB (the unit to be reduced by purification) is present in YNL (neutral lactase solution) was considered. Specifically, PrB with a predetermined amount of protein (0.0113, 0.113, 1.13, 11.3 or 113 ng) was added to unfragmented lactase (F-1) to react at 30°C for 20 hours, and the states of fragmentation were confirmed by SDS-PAGE. The results are shown in Fig. 6. From these states of fragmentation, fragmentation was not caused when 0.26 ng {corresponding to a protein amount of 11.3 ng mentioned above (Lane 4)} was used with respect to 1 NLU of lactase activity (fractions after fragmentation were not detected by the above-described Image J software).

### 4. Effect of Removing PrB Using Resin Column Chromatography

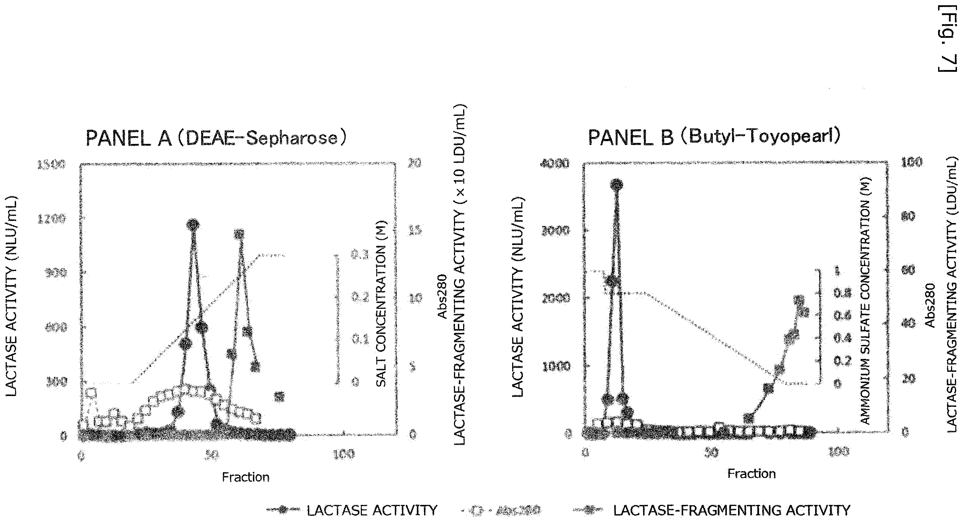

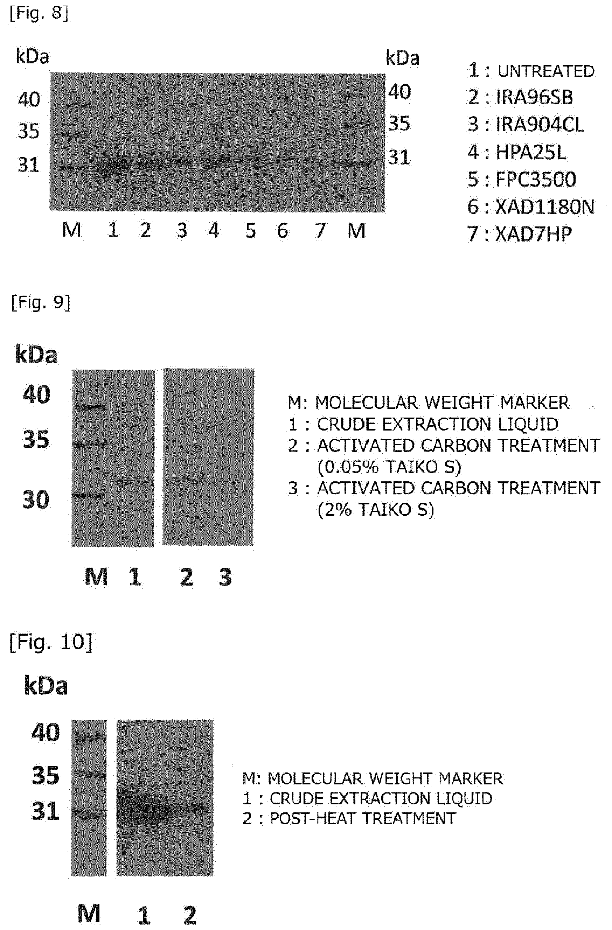

When a crude extraction liquid (including 200 LDU/mL of PrB) obtained by carrying out ultrasonic treatment and removing the insoluble fraction by centrifugal separation was applied to DEAE-Sepharose (weakly basic anion-exchange column chromatography) and Butyl-Toyopearl (hydrophobic interaction column chromatography), lactase and PrB could be separated even when using either resin. The states of separation were shown in Figs. 7 and 8. As shown in Table 3, the recoveries of lactase activity in the fractions obtained by DEAE-Sepharose and Butyl-Toyopearl column chromatography were 104% and 95%, respectively. In addition, the lactase-fragmenting activity of the collected lactase fractions was examined, and the results were below the detection limit. These were applied to the heat stability test (the conditions are the same as above), and the results were about 45% and about 44%, respectively, which were higher than those of the above-described YNL B (not shown).

### [Table 3]

**[Table 3] WHOLE ACTIVITY AND RECOVERY OF ACTIVITY OF LACTASE FRACTION COLLECTED BY EACH CHROMATOGRAPHY**

| | WHOLE ACTIVITY (x10³ NLU) | RECOVERY OF ACTIVITY (%) |
|---|---|---|
| DEAE-Sepharose Fraction | 28.8 | 104 |
| Butyl- Toyopearl Fraction | 28.3 | 95 |

### 5. Effect of Removing PrB by Mixing with Resin

Industrial resins and a crude extraction liquid obtained in the same manner as above were mixed to react. The supernatant was collected after completion of the reaction, and lactase activity was measured. Subsequently, PrB was detected by Western blotting. The results are shown in Fig. 8 and Table 4. From the results, it was found that PrB could be removed using a synthetic absorbent, XAD7HP.

### [Table 4]

**[Table 4] CHANGES IN PrB RESIDUAL RATE BY REACTION WITH INDUSTRIAL RESIN**

| RESIN | BAND AREA | PrB PROTEIN AMOUNT PER LANE (ng/Lane) | PrB RESIDUAL RATE (%) |
|---|---|---|---|
| UNTREATED | 9214 | 23.6 | 100 |
| IRA96SB | 4156 | 17.9 | 76 |
| IRA904CL | 2539 | 15.3 | 65 |
| HPA25L | 1409 | 12.8 | 54 |
| FPL3500 | 1087 | 11.8 | 50 |
| XAD1180N | 547 | 9.7 | 41 |
| XAD7HP | 71 | 5.4 | 23 |

### 6. Effect of Removing PrB by Activated Carbon Treatment

A sample (1) in which TAIKO S was added to a crude extraction liquid obtained in the same manner as above at a final concentration of 0.05%, and a sample (2) in which TAIKO S was added thereto at a final concentration of 2% were prepared and used as liquids treated with activated carbon. The supernatant was collected, and PrB was detected by Western blotting. The results of PrB detection by Western blotting are shown in Fig. 9, and the results of calculated PrB residual rate are shown in Table 5. As shown in the results, PrB could be removed by adding activated carbon in an amount of not less than 0.05%.

### [Table 5]

**[Table 5] CHANGES IN PrB RESIDUAL RATE BY ACTIVATED CARBON**

| SAMPLE | BAND AREA | PrB PROTEIN AMOUNT PER LANE (ng/Lane) | PrB RESIDUAL RATE (%) |
|---|---|---|---|
| CRUDE EXTRACTION LIQUID | 7125 | 21.5 | 100 |
| ACTIVATED CARBON (0.05% TAIKO S) | 4485 | 18.3 | 85 |
| ACTIVATED CARBON (2% TAIKO S) | 0 | < 1.57 | <7 |

### 7. Reduction in PrB Before and After Heat Treatment

A crude extraction liquid obtained in the same manner as above was used as a sample, and PrB was detected by Western blotting. Consequently, PrB was significantly reduced by heat treatment (43°C, 2 hours). The results are shown in Fig. 10 and Table 6. It should be noted that, when the amount of PrB protein before heat treatment is regarded as 100%, the amount of PrB protein after heat treatment was calculated as a % value as the PrB residual rate.

### [Table 6]

**[Table 6] CHANGES IN PrB RESIDUAL RATE BY HEAT TREATMENT**

| SAMPLE | BAND AREA | PrB PROTEIN AMOUNT PER LANE (ng/Lane) | PrB RESIDUAL RATE (%) |
|---|---|---|---|
| CRUDE EXTRACTION LIQUID | 20363 | 34.5 | 100 |
| POST-HEAT TREATMENT (43°C, 2hr) | 4469 | 18.3 | 53 |

A commercial available neutral lactase solution (YNL A) was used as a sample, and PrB was detected by Western blotting. Consequently, the YNL solution contained 11.5 ng/NLU as PrB per NLU of lactase activity. The results are shown in Table 7.

### [Table 7]

**[Table 7] PrB CONCENTRATION IN LACTASE PREPARATION**

| SAMPLE | BAND AREA | PrB PROTEIN AMOUNT PER LANE (ng/Lane) | PrB PER NLU of LACTASE (ng/NLU) |
|---|---|---|---|
| YNL A | 9027 | 23.4 | 11.5 |

### Sequence listing

GODO1-9_ST25.txt

## Claims

1. Proteinase B comprising the following enzymatic properties,
1) having an optimum temperature of about 40°C,
2) having an optimum pH of about 8.0,
3) being stable at pH 5.0 to 8.0,
4) having high substrate specificity to FITC-casein and lactase,
5) having a neutral lactase-fragmenting action, and
6) having a molecular weight of about 29,700 to 30,000 (by SDS-PAGE).

2. The proteinase B according to claim 1, satisfying the following (a) or (b),
(a) a polypeptide whose amino acid sequence is represented by SEQ ID NO:1 in the sequence listing, and
(b) a polypeptide derived from the polypeptide of (a) by deletion, substitution or addition of one or several amino acid residues in the amino acid sequence represented by SEQ ID NO:1 in the sequence listing, wherein the amino acid sequence has a homology of not less than 70% to the sequence of SEQ ID NO:1, and having a neutral lactase-fragmenting action at pH 5.0 to 8.0.

3. A precursor of proteinase B, satisfying the following (a) or (b),
(a) a polypeptide whose amino acid sequence is represented by SEQ ID NO:2 in the sequence listing, and
(b) a polypeptide derived from the polypeptide of (a) by deletion, substitution or addition of one or several amino acid residues in the amino acid sequence represented by SEQ ID NO:2 in the sequence listing, wherein the amino acid sequence has a homology of not less than 70% to the sequence of SEQ ID NO:1, and its mature protein is a polypeptide having a neutral lactase-fragmenting action at pH 5.0 to 8.0.

4. Apolynucleotide coding for an amino acid sequence forming proteinase B according to claim 1 or 2, or the precursor of proteinase B according to claim 3.

5. The polynucleotide according to claim 4, selected from the group consisting of the following (A) to (D),
(A) a polynucleotide whose nucleotide sequence is represented by SEQ ID NO:3 in the sequence listing, or
(B) a polynucleotide which hybridizes with the polynucleotide whose nucleotide sequence is represented by SEQ ID NO:3 in the sequence listing under stringent condition, and which codes for a polypeptide having a neutral lactase-fragmenting action at pH 5.0 to 8.0,
(C) a polynucleotide whose nucleotide sequence is represented by SEQ ID NO:4 in the sequence listing, or
(D) a polynucleotide which hybridizes with the polynucleotide whose nucleotide sequence is represented by SEQ ID NO:4 in the sequence listing under stringent condition, and which codes for a polypeptide comprising an amino acid sequence whose mature protein is a polypeptide having a neutral lactase-fragmenting action at pH 5.0 to 8.0.

6. A lactase solution comprising less than 11.5 ng of proteinase B according to claim 1 or 2 per unit of neutral lactase activity.

7. The lactase solution according to claim 6, comprising not less than 0.01 ng of proteinase B according to claim 1 or 2 per unit of neutral lactase activity.

8. The lactase solution according to claim 6 or 7, which comprises a protease inhibitor.

9. The lactase solution according to any one of claims 6 to 8, which comprises a stabilizer.

10. The lactase solution according to any one of claims 6 to 9, for use in UHT milk or yogurt.

11. A method for producing a lactase solution according to any one of claims 6 to 10,
the method comprising the steps of removing the proteinase B from the lactase solution and/or reducing the action of the proteinase B.

12. The method for producing a lactase solution according to claim 11, wherein the step of removing the proteinase B is carried out by weakly basic anion-exchange column chromatography or hydrophobic interaction chromatography to the lactase solution.

13. The method for producing a lactase solution according to claim 11, wherein the step of removing the proteinase B is carried out by treating the lactase solution with activated carbon.

14. The method for producing a lactase solution according to any one of claims 11 to 13, wherein the step of reducing the action of the proteinase B is carried out by treating the lactase solution with heat.

15. A method for evaluating heat stability of lactase comprising measuring an amount of the proteinase B protein according to claim 1 or 2 and a lactase activity thereof, contained in a lactase solution, and using the amount of proteinase B protein per unit of lactase activity as an index.

16. An antibody that binds specifically to the polypeptide whose amino acid sequence is represented by SEQ ID No.1.

17. The antibody according to claim 16, which is a polyclonal antibody against the peptide whose amino acid sequence is a portion of the amino acid sequence represented by SEQ ID No.1, from position 27 to position 45.
